(19) Europäisches Patentamt
European Patent Office
Office européen des brevets

(11) Numéro de publication: **0 025 765**
**B1**

(12) **FASCICULE DE BREVET EUROPEEN**

(45) Date de publication du fascicule du brevet: **22.12.82**

(21) Numéro de dépôt: **80401314.2**

(22) Date de dépôt: **12.09.80**

(51) Int. Cl.³: **C 07 C 11/12, C 07 C 15/00, C 07 C 7/10, C 07 C 7/08, C 10 G 21/22**

(54) **Procédé de séparation, à l'aide de sulfonamides, d'hydrocarbures diéniques et/ou aromatiques de coupes d'hydrocarbures les contenant.**

(30) Priorité: **14.09.79 FR 7923041**

(43) Date de publication de la demande:
**25.03.81 Bulletin 81/12**

(45) Mention de la délivrance du brevet:
**22.12.82 Bulletin 82/51**

(84) Etats contractants désignés:
**AT BE DE GB IT NL**

(56) Documents cités:
**BE - A - 871 447**
**FR - A - 2 388 874**

(73) Titulaire: **COMPAGNIE FRANCAISE DE RAFFINAGE Société anonyme dite:**
**5, rue Michel-Ange**
**F-75781 Paris Cedex 16 (FR)**

(73) Titulaire: **ANVAR Agence Nationale de Valorisation de la Recherche**
**43, rue Caumartin**
**F-75436 Paris Cedex 09 (FR)**

(72) Inventeur: **Atlani, Martial**
**52 rue Bouret**
**F-75019 Paris (FR)**
Inventeur: **Loutaty, Roben**
**29, rue Séry**
**F-76600 Le Havre (FR)**
Inventeur: **Wakselman, Claude**
**18, rue du Clos d'Alençon**
**F-91120 Villebon sur Yvette (FR)**
Inventeur: **Yacono, Charles**
**68, rue Armand Barbès**
**F-76600 Le Havre (FR)**

(74) Mandataire: **Jolly, Jean-Pierre et al,**
**Cabinet BROT 83, rue d'Amsterdam**
**F-75008 Paris (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

EP 0 025 765 B1

Procédé de séparation, à l'aide de sulfonamides, d'hydrocarbures diéniques et/ou aromatiques de coupes d'hydrocarbures les contenant.

La présente invention concerne la séparation, à l'aide de sulfonamides, d'hydrocarbures diéniques et/ou aromatiques de coupes d'hydrocarbures les contenant; elles concerne plus particulièrement l'utilisation, en vue de cette séparation, dans les techniques connues d'extraction liquide-liquide et de distillation extractive, de solvants choisis dans le groupe constitué par les sulfonamides.

Dans la suite de la présente description, on entendra par sulfonamides des composés comportant dans leur structure moléculaire le groupement

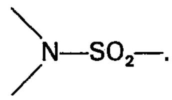

De mobreux solvants sélectifs de récupération d'hydrocarbures sont décrits dans la littérature technique. Concernant les hydrocarbures diéniques, on peut citer particulièrement la N-méthylpyrrolidone, l'acétonitrile et la diméthylformamide. Une des applications possibles de ces solvants est, par exemple, l'extraction sélective de l'isoprène d'une coupe d'hydrocarbures dont le nombre d'atomes de carbone est égal ou voisin de 5 (cette coupe d'hydrocarbures est communément appelée "coupe $C_5$").

Parmi les solvants sélectifs connus pour extraire les hydrocarbures aromatiques, on peut citer le solfolane, la N-méthylpyrrolidone en solution aqueuse, des dérivés de la morpholine tels que la N-formylmorpholine, ou le diméthylsufoxyde.

Il est également connu d'utiliser certaines sulfonamides aliphatiques saturées pour séparer les hydrocarbures diéniques et/ou aromatiques.

Dans la demande de brevet français n° 78 29828, du 19 Octobre 1978, dont les Demanderesses sont titulaires, celles-ci ont décrit, pour une telle utilisation, des sulfonamides de formule générale:

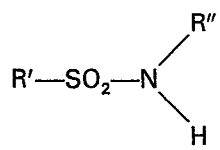 ,

où R′ et R″ sont des restes alkyle saturés possédant de 1 à 4 atomes de carbone.

Dans le brevet BE 871 447, qui appartient également aux Demanderesses, celles-ci ont décrit un procédé de séparation similaire utilisant des sulfonamides, mono ou disubstituées sur l'azote, notamment par des radicaux alkyle.

Des sulfonamides du type

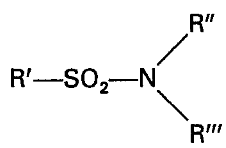 ,

où R′, R″ et R‴ sont des restes alkyle saturés possédant de 1 à 4 atomes de carbone, sont décrites pour le même utilisation dans la demande de brevet français n° 2 388 874 et dont les Demanderesses sont également titulaires.

Les Demanderesses ont établi maintenant qu'il est également possible d'utiliser, comme solvants d'extraction liquide-liquide et/ou de distillation extractive pour séparer des hydrocarbures diéniques et/ou aromatiques de coupes d'hydrocarbures les contenant, des sulfonamides de formule générale analogue à celles utilisées selon FR—A—2 388 874, mais dans lesquelles un au moins des restes alkyle est insaturé.

La présente invention a par conséquent pour objet un procédé d'extraction d'hydrocarbures diéniques et/ou aromatiques de coupes d'hydrocarbures les contenant, par les techniques connues d'extraction liquide-liquide et/ou de distillation extractive, ledit procédé étant caractérisé en ce qu'on emploie comme solvant, éventuellement en mélange avec un ou plusieurs autres corps, au moins une sulfonamide choisie dans le groupe constitué par les composés de formule générale:

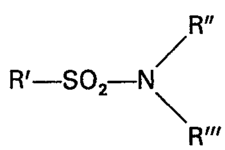

où R′, R″ et R‴ sont des restes alkyle, linéaires ou ramifiés, possédant de 1 à 18 atomes de carbone, où deux ou trois des restes R′, R″ et R‴ peuvent être identiques, où l'un des restes R″ ou R‴ peut être remplacé par un atome d'hydrogène, et où au moins l'un des restes R′, R″, et R‴ est insaturé.

Les Demanderesses ont ainsi utilisé avec succès:

— la N,N diallyl méthanesulfonamide

— et la N,N méthyl allylméthanesulfonamide.

Les Demanderesses ont établi l'efficacité de ces sulfonamides dans un procédé d'extraction des hydrocarbures diéniques et/ou aromatiques à partir de coupes qui les contiennent. Cette efficacité peut être mise en évidence par plusieurs tests: ou peut mesurer la sélectivité à dilution infinie du solvant considéré vis-à-vis d'un couple d'hydrocarbures témoin.

On peut aussi réaliser l'extraction par une méthode classique et analyser l'extrait, d'une part, le raffinat, d'autre part. De tels tests seront rapportés ci-après dans des exemples.

Quelle que soit la technique d'extraction utilisée, les quantités de solvant employées sont, en général, comprises entre 0,5 et 5 fois le volume de la charge d'hydrocarbures à traiter, mais cette quantité peut être supérieure, notamment si l'on désire réduire le nombre d'étages théoriques nécessaires à l'extraction.

Le solvant utilisé peut être plus ou moins pur: la pureté dite "commerciale" convient le plus souvent pour réaliser l'extraction. Les sulfonamides peuvent être utilisées telles quelles, en mélange entre elles ou avec d'autres sulfonamides, ou encore en mélange avec un ou plusieurs autres corps. L'extraction peut être effectuée par extraction liquide-liquide ou par distillation extractive.

Les deux techniques peuvent d'ailleurs être combinées, la charge étant enrichie, dans une première étape, en espèce chimique à extraire, par extraction liquide-liquide, puis, dans une seconde étape, soumise à une distillation extractive, qui permet de récupérer l'espèce chimique recherchée.

Trois figures sont jointes, à titre d'illustrations non limitatives, à la présente description.

Les figures 1 et 2 sont des schémas illustrant l'application du procédé selon l'invention à l'extraction en continu de l'isoprène d'une charge d'hydrocarbures contenant cinq atomes de carbone.

La figure 3 est un schéma illustrant l'application du procédé selon l'invention à l'extraction en continu des hydrocarbures aromatiques d'une charge et contenant.

Sur ces figures, les recycles de tête et de fond des collones n'ont pas été représentés, par souci de clarté.

On se réfèrera d'abord à la figure 1.

Une charge, obtenue par distillation d'essence de craquage et ayant été éventuellement soumise au préalable à un traitement thermique destiné à dimériser le cyclopentadiène, est introduite par la ligne 1, à l'état liquide, dans la partie inférieure d'une colonne d'extraction liquide-liquide 2. Le solvant, contenant au moins une sulfonamide, est introduit par la ligne 3, à l'état liquide, dans la partie supérieure de la colonne 2.

On recueille par la ligne 4, du fond de la colonne 2, le solvant contenant les dioléfines et une petite quantité de pantènes.

On recueille par la ligne 5, au sommet de la colonne 2, le raffinat constitué principalement par les pentanes, les pentènes et de légères traces de diènes. Ce raffinat est conduit par la ligne 5 dans une enceinte de distillation 6, où il est séparé des traces de solvant qu'il contient. Le raffinat est évacué de l'enceinte 6 par la ligne 7, tandis que le solvant est conduit à la ligne 4 par la ligne 8.

Le solvant contenant les dioléfines et une petite quantité de pentènes circulant dans la ligne 4 est conduit dans la partie supérieure d'une colonne de rectification 9.

Les vapeurs recueillies en tête de colonne 9 par la ligne 10 sont liquéfiées dans le réfrigérant 11; le liquide résultant est introduit par la ligne 12 dans la partie inférieure de la colonne 2. Le solvant, chargé en dioléfines, en oléfines et en hydrocarbures acétyléniques, est introduit par la ligne 13 dans la partie médiane de la colonne de distillation extractive 14. En tête de la colonne 14, on recueille, par la ligne 15, un mélange d'isoprène et de cyclopentène, que l'on sépare par distillation dans les colonnes 16 et 17, l'isoprène étant recueilli par la ligne 18 en tête de la colonne 16, et le cyclopentène, par un soutirage latéral de la colonne 17, par la ligne 19. Le fond, recueilli dans la ligne 20, et/ou la tête, recueillie dans la ligne 21 de la colonne 17, peuvent être recyclés dans la charge de la colonne 2.

Par la ligne 22, on soutire latéralement de la colonne 14 un courant gazeux comprenant principalement les pentadiènes, le cyclopentadiène et les hydrocarbures acétyléniques. Les vapeurs de solvant sont séparées des hydrocarbures par distillation dans l'enceinte 23.

Les hydrocarbures sont recueillis au sommet de l'enceinte 23 par la ligne 24. Le solvant recueilli dans le fond de l'enceinte 23 par la ligne 25 est recyclé dans la colonne 2 par la ligne 3. Du solvant frais peut être introduit dans la ligne 25 par la ligne 26 et du solvant usé peut être évacué par la purge 27.

Dans une autre forme de mise en oeuvre représentée schématiquement sur la figure 2, pour laquelle les éléments identiques à ceux de la figure 1 ont été identifiés par les mêmes références affectées de l'indice ′, la colonne d'extraction 2′ est identique à la colonne 2 de la figure 1, mais la colonne de rectification 30 est plus efficace que la colonne 9, ce qui permet de prélever latéralement, par la ligne 31, un mélange de vapeurs d'isoprène et de cyclopentène chargées en solvant et, en fond, par la ligne 32, la majeure partie du solvant liquide chargé des hydrocarbures moins volatils en présence du solvant, c'est-à-dire le cyclopentadiène, les pentadiènes et les hydrocarbures acétyléniques. Les vapeurs de solvant sont séparées de l'isoprène et du cyclopentène par distillation dans l'enceinte 33.

L'isoprène et le cyclopentène sont recueillis par la ligne 34, le solvant est renvoyé dans la colonne 30 par la ligne 35. Le fond de la colonne 30 est envoyé par la ligne 32 dans un dispositif de récupération du solvant constitué par une colonne 36; le solvant recueilli par la ligne 37, au fond de la colonne 36, est recyclé dans la colonne 2' par la ligne 3'; la colonne 36 comporte un soutirage latéral 38, par lequel est prélevé un mélange de cyclopentadiène, de pentadiènes et d'hydrocarbures acétyléniques. En tête de la colonne 36 est recueillie une phase vapeur, par la ligne 39, qui est recyclée dans la partie inférieure de la colonne 30. La ligne 34 alimente une colonne de fractionnement 40, de laquelle on recueille l'isoprène, en tête, par la ligne 41, et le cyclopentène, au fond, par la ligne 42. Du solvant frais peut être introduit dans la ligne 37 par la ligne 44 et du solvant usé introduit dans la ligne 37 par la ligne 44 et du solvant usé peut être évacué par la purge 43.

Dans le cas de la figure 3, une charge, dont on veut séparer les hydrocarbures aromatiques, obtenue par reformage d'une coupe pétrolière, est introduite par la ligne 51 dans la partie médiane d'une colonne d'extraction 52. Le solvant, contenant an moins une sulfonamide, est introduit par la ligne 53 à l'état liquide dans la partie supérieure de la colonne 52. On recueille par la ligne 54, au fond de la colonne 52, le solvant contenant les hydrocarbures aromatiques et une certaine quantité d'hydrocarbures non aromatiques. On recueille par la ligne 55, au sommet de la colonne 52, le raffinat constitué par des hydrocarbures non aromatiques.

Ce raffinat est conduit par la ligne 55 dans l'enceinte de distillation 56, où il est séparé des traces de solvant qu'il contient. Le raffinat est évacué de l'enceinte 56 par la ligne 57. Le solvant recueilli par la ligne 58 est recyclé à la colonne 52 par la ligne 53, après passage dans un réfrigérant 59. Le solvant contenant les hydrocarbures aromatiques et une certaine quantité d'hydrocarbures non aromatiques recueillie par la ligne 54 est conduit dans la partie médiane d'une colonne de distillation extractive 60. En tête de la colonne 60, on recueille par la ligne 61 les hydrocarbures non aromatiques et une petite quantité d'hydrocarbures aromatiques qui sont recyclés dans le bas de la colonne 52. On recueille par la ligne 62, dans le fond de la colonne 60, le solvant contenant les hydrocarbures aromatiques. Le solvant contenant les hydrocarbures aromatiques est conduit dans la partie médiane d'une colonne 63 fonctionnant sous pression réduite. On recueille dans le fond de la colonne 63, par la ligne 64, le solvant qui est recycle à la colonne 52.

On recueille au sommet de la colonne 63, par la ligne 65, les hydrocarbures aromatiques contenant des traces de solvant, qui sont séparées dans l'enceinte de distillation 66. On recueille au fond de l'enceinte 66, par la ligne 67, le solvant, qui est recyclé à la colonne 52. Les hydrocarbures aromatiques sont recueillis au sommet de l'enceinte 66 par la ligne 68.

L'invention est illustrée, en outre, par les exemples qui suivent et qui n'ont aucun caractère limitatif.

Les exemples 1 et 2 concernent l'utilisation de sulfonamides selon l'invention pour l'extraction d'hydrocarbures aromatiques d'un mélange en contenant.

L'exemple 3 concerne l'utilisation de sulfonamides selon l'invention pour l'extraction d'hydrocarbures diéniques d'un mélange en contenant.

## Exemple 1

Cet exemple illustre l'utilisation de solvants selon l'invention pour l'extraction d'hydrocarbures aromatiques de mélanges d'hydrocarbures les contenant, par distillation extractive, ou par extraction liquide-liquide, ou par une combinaison de ces deux techniques.

On a déterminé, pour deux solvants selon l'invention, à savoir la N,N méthyl allyl méthanesulfonamide et la N,N diallyl méthanesulfonamide, la sélectivité à dilution infinie à 50°C vis-à-vis de deux couples d'hydrocarbures ayant des points d'ébullition voisins, le couple benzène-2,4-diméthylpentane et le couple benzène-cyclohexane.

La sélectivité à dilution infinie d'un solvant vis-à-vis d'une couple d'hydrocarbures que l'on se propose de séparer par distillation extractive ou par extraction liquide-liquide, se définit comme le rapport des coefficients d'activité à dilution infinie des deux hydrocarbures dans ce même solvant. La séparation est d'autant plus efficace que la sélectivité à dilution infinie est plus élevée (voir "Propriétés thermodynamiques des solutions infiniment diluées d'hydrocarbures dans les solvant polaires" par P. VERNIER, C. RAIMBAULT et H. RENON, Journal de Chimie Physique, 1969, v. 66, nº 3, pages 429 à 436).

Les coefficients d'activité à dilution infinie ont été mesurés par le méthode du "diluteur exponentiel" (voir "Accurate measurement of activity coefficients at infinite dilution by insert gas stripping and gas chromatography" par J. C. LEROI, J. C. MASSON, H. RENON, J. F. FABRIES et H. SANNIER, Ind. Eng. Chem. Process Des. Dev. V. 16, nº 1, pages 139 à 144, 1977).

Les résultats sont donnés dans le Tableau 1 suivant.

TABLEAU 1

| Hydrocarbures | | Coefficients d'activité à dilution infinie à 50°C | | Sélectivités à dilution infinie à 50°C par rapport au benzène | |
|---|---|---|---|---|---|
| Nom | Température sous 760 mm Hg (°C) | dans la N,N-méthyl allyl méthane-sulfonamide | dans la N,N% diallyl méthane-sulfonamide | dans la N,N-méthyl méthane sulfonamide | dans la N,N-diallyl méthane-sulfonamide |
| Benzène | 80,1 | 1,37 | 1,04 | 1 | 1 |
| Cyclohexane | 80,7 | 6,76 | 4,79 | 4,93 | 4,61 |
| 2,4,diméthyl-pentane | 80,5 | 9,85 | 8,95 | 7,19 | 8,61 |

Les valeurs obtenues pour les sélectivités à dilution infinie montrent que les séparations sont efficaces. On peut donc extraire le benzène de ses mélanges avec le diméthyl 2—4 pentane ou avec le cyclohexane en utilisant les solvants selon l'invention.

Exemple 2

Cet exemple concerne l'utilisation de solvants selon l'invention pour l'extraction d'un hydrocarbure aromatique par extraction liquide-liquide. On prépare dans une ampoule à décanter un mélange ternaire de benzène (ci-après dénommé le soluté), de n-heptane (ci-après dénommé le diluant) et d'un solvant selon l'invention.

Après agitation, on laisse reposer à la température de 20°C. On obtient ainsi deux phases. Les résultats de l'essai effectué figurent dans le Tableau 2 ci-après où:

— le coefficient de partage est le rapport:

$$\frac{\text{fraction massique HC (soluté ou diluant) dans l'extrait}}{\text{fraction massique du même HC dans le raffinat}}$$

HC signifiant hydrocarbure,

— le facteur de séparation est le rapport:

$$\frac{\text{coefficient de partage du soluté}}{\text{coefficient de partage du diluant}}$$

Les fractions massiques du mélange ternaire ont été déterminées par pesée, celles de l'extrait et du raffinat par chromatographie en phase gazeuse.

TABLEAU 2

| Mélange ternaire à 20°C | Composition du mélange en % poids | Composition de l'extrait en % poids | Composition du raffinat en % poids | Coefficients de partage | Facteur de séparation |
|---|---|---|---|---|---|
| benzène | 17,60 | 16,26 | 20,91 | 0,778 | 8,47 |
| n-neptane | 32,80 | 6,94 | 75,59 | 0,092 | |
| N,N-méthyl allyl méthanesulfonamide | 49,60 | 76,80 | 3,50 | | |

Les valeurs obtenues pour les facteurs de séparation montrent que les séparations sont efficaces. On peut donc extraire le benzène de son mélange avec le n-heptane à l'aide de solvants selon l'invention.

Exemple 3

Cet exemple concerne l'utilisation de solvants selon l'invention, pour l'extraction d'hydrocarbures diéniques de mélanges d'hydrocarbures les contenant, par distillation extractive, ou par extraction liquide-liquide, ou par une combinaison de ces deux techniques.

On a déterminé, pour deux solvants selon l'invention, la sélectivité à dilution infinie à 30°C vis-à-vis de deux couples d'hydrocarbures ayant des points d'ébullition voisins, le couple isoprène-n pentane et le couple isoprèneméthyl-2 butène-2.

La sélectivité à dilution infinie est définie et déterminée de la même façon que pour l'exemple 1.

Les résultats sont donnés dans le Tableau 3 ci-après.

TABLEAU 3

| Hydrocarbures | | Coefficients d'activité à dilution infinie à 30°C | | Sélectivités à dilution infinie à 30°C par rapport à l'isoprène | |
|---|---|---|---|---|---|
| Nom | Température d'ébullition sous 760 mm Hg (°C) | dans la N,N-méthyl allyl méthane-sulfonamide | dans la N,N-diallyl méthane-sulfonamide | dans la N,N-méthyl allyl méthane-sulfonamide | dans la N,N-diallyl méthane-sulfonamide |
| Isoprène | 34,1 | 3,03 | 2,03 | 1 | 1 |
| Méthyl-2 butène-2 | 38,6 | 5,69 | 3,43 | 1,88 | 1,69 |
| n-pentane | 36,1 | 11,88 | 6,73 | 3,92 | 3,31 |

Les valeurs obtenus pour les sélectivités à dilution infinie montrent que les séparations sont efficaces. On peut donc extraire l'isoprène de ses mélanges avec le n-pentane ou avec le méthyl-2 butène-2 en utilisant les solvants selon l'invention.

## Revendications

1. Procédé d'extraction d'hydrocarbures diéniques et/ou aromatiques de coupes d'hydrocarbures les contenant, par les techniques connues d'extraction liquide-liquide et/ou de distillation extractive, ledit procédé étant caractérisé en ce qu'on emploie comme solvant, éventuellement en mélange avec un ou plusieurs autres corps, au moins une sulfonamide choisie dans le groupe constitué par les composés de formule générale:

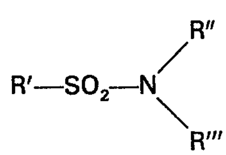
,

où R', R'' et R''' sont des restes alkyle, linéaires ou ramifiés, possédant de 1 à 18 atomes de carbone, où deux ou trois des restes R', R'' et R''' peuvent être identiques, où l'un des restes R'' ou R''' peut être remplacé par un atome d'hydrogène, et où au moins l'un des restes R', R'' et R''' est insaturé.

2. Procédé selon la revendication 1, caractérisé en ce que la sulfonamide est choisie dans le groupe constitué par la N,N,diallyl méthanesulfonamide et la N,N,méthyl allyl méthanesulfonamide.

## Patentansprüche

1. Verfahren zur Extraktion von Dienen und/oder aromatischen Kohlenwasserstoffen aus dieselben enthaltenden Kohlenwasserstoffchargen mittels bekannter Flüssig/Flüssig-Extraktion und/oder extraktiver Destillation, dadurch gekennzeichnet, daß als Lösungsmittel, gebenenfalls in Mischung mit einem oder mehreren anderen Körpern, mindestens ein Sulfonamid aus der Gruppe der

Verbindungen der allgemeinen Formel

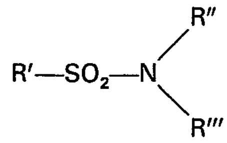

verwendet wird, wobei R', R″ und R‴ geradkettige oder verzweigte Alkylreste mit 1 bis 18 C-Atomen sind, zwei oder drei dieser Reste R', R″ und R‴ identisch sein können, einer der Reste R″ und R‴ durch ein Wasserstoffatom ersetzt sein kann und mindestens einer der Reste R', R″ und R‴ ungesättigt ist.

2. Verfahren nach Anspruch 1, dadurch gekennzeichnet, daß das Sulfonamid aus der Gruppe: N,N-Diallyl-methansulfonamid und N,N-Methyl-allyl-methansulfonamid, ausgewählt wird.

## Claims

1. A process for extracting dienic and/or aromatic hydrocarbons from hydrocarbon samples containing them, by known liquid-liquid extraction techniques and/or extractive distillation, said process being characterized in that there is used as solvent, possible mixed with one or more other substances, at least one sulfonamide chosen from the group formed by the compounds having the general formula:

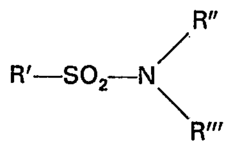

in which R', R″ and R‴ are linear or branched alkyl remainders having from 1 to 18 carbon atoms, in which two or three of the remainders R', R″ and R‴ may be identical, in which one of the remainders R″ or R‴ may be replaced by a hydrogen atom and in which at least one of the remainders R', R″ and R‴ is unsaturated.

2. The process according to claim 1, characterized in that the sulfonamide is chosen from the group formed by N,N,diallyl methanesulfonamide and N,N,methyl allyl methanesulfonamide.

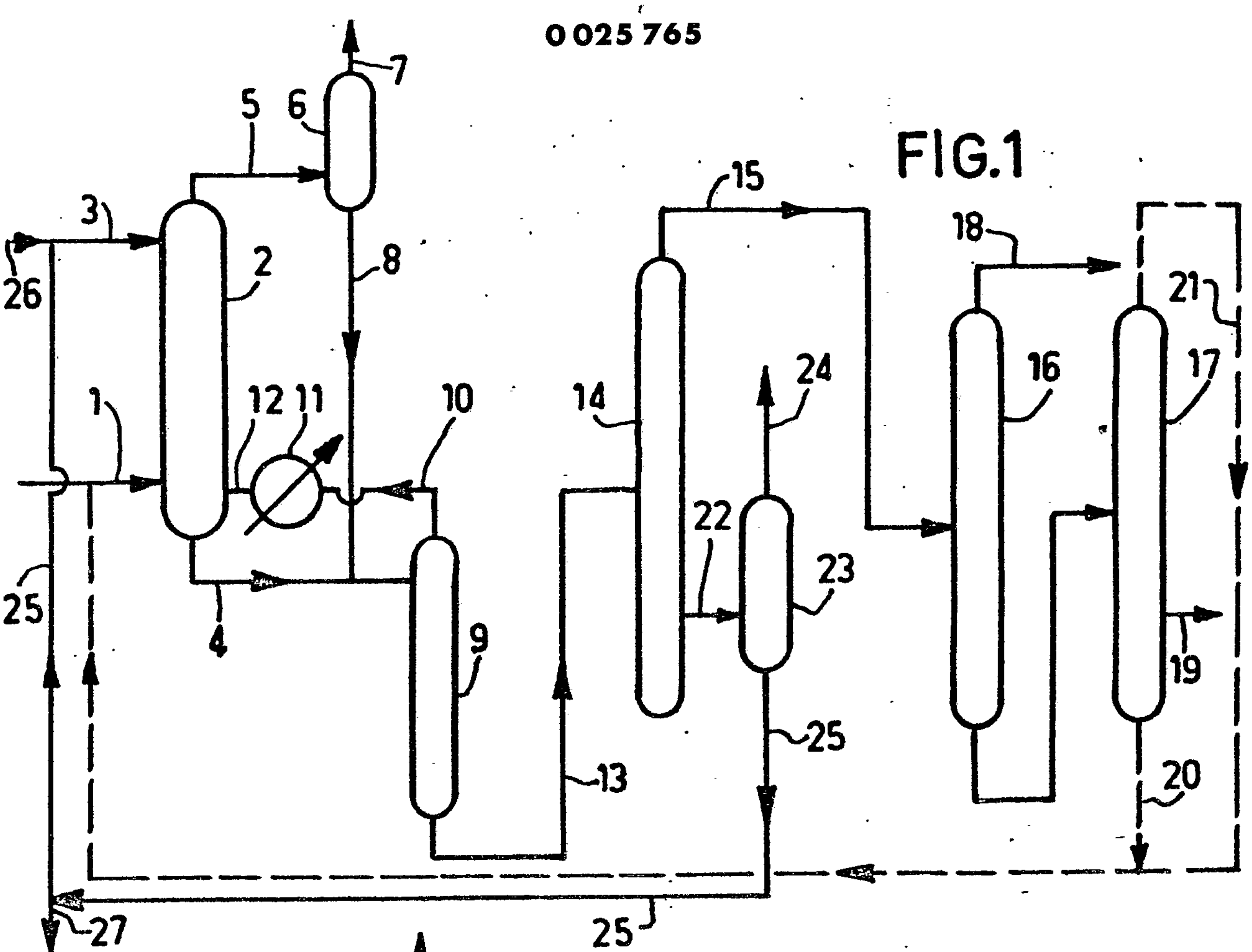

1
2
3
4
5
6
7
8
9
10
11
12
13
14
15
16
17
18
19
20
21
22
23
24
25
26
27


FIG.1

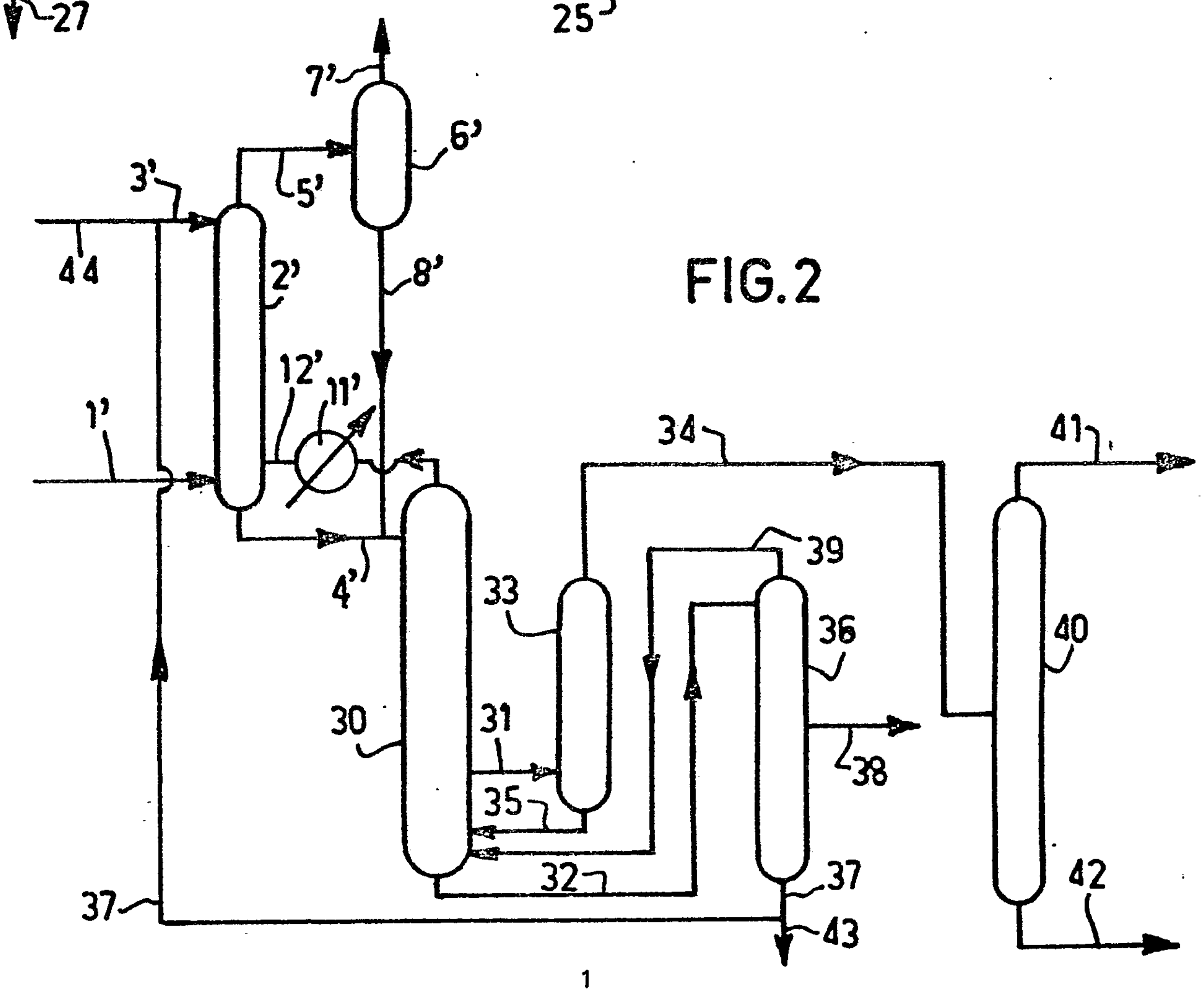

1'
2'
3'
4'
5'
6'
7'
8'
11'
12'
30
31
32
33
34
35
36
37
38
39
40
41
42
43
44


FIG.2

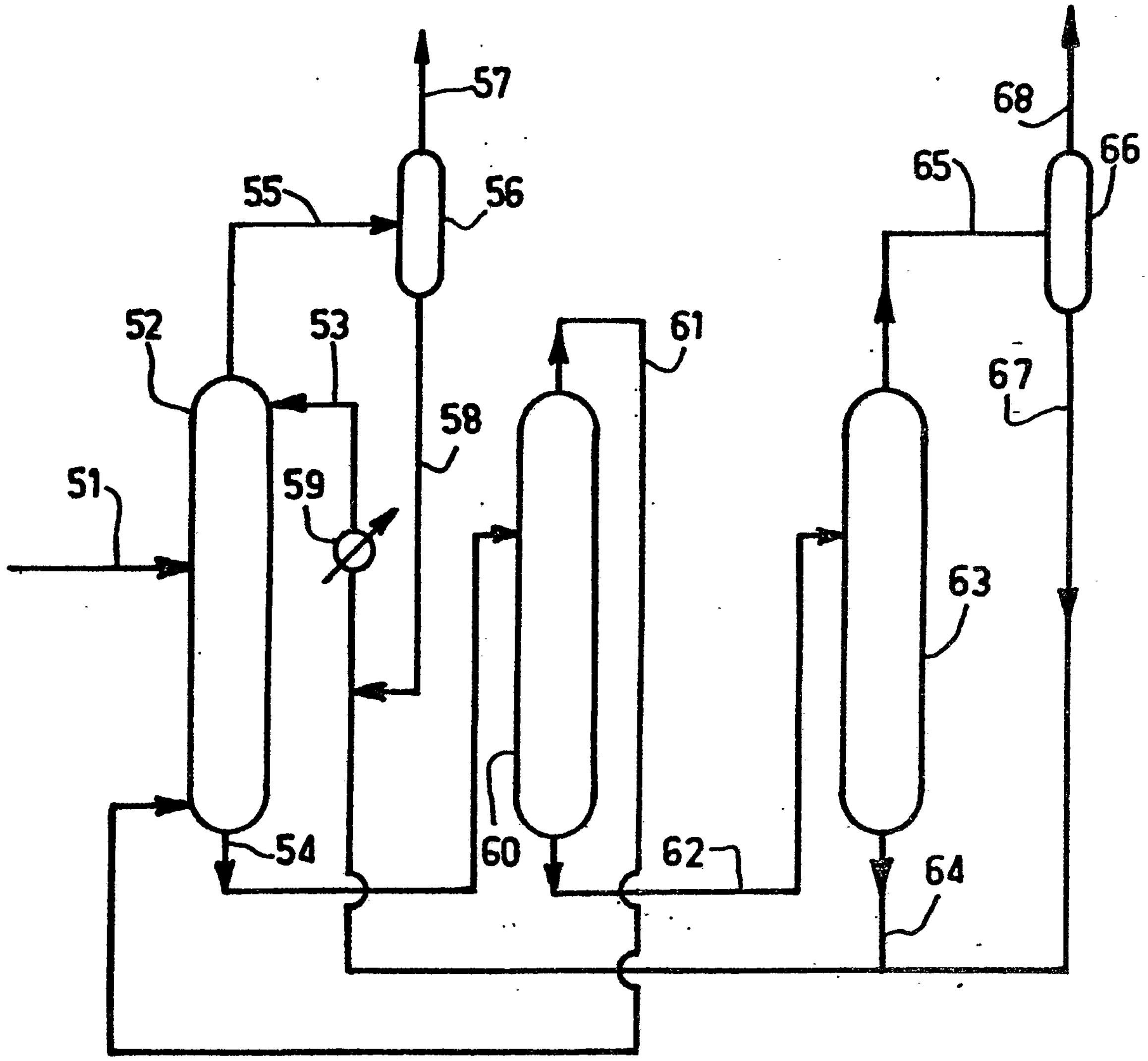


FIG.3